# EUROPÄISCHE PATENTSCHRIFT

(11) **EP 4 601 588 B1**
(45) Veröffentlichungstag und Bekanntmachung des Hinweises auf die Patenterteilung: **11.02.2026**
(21) Anmeldenummer: 23789282.3
(22) Anmeldetag: 09.10.2023
(51) Int. Cl.: A61F 2/70, A61F 2/66, A61F 2/68

(54) **VERFAHREN ZUM STEUERN EINER UNTERSCHENKELPROTHESE UND UNTERSCHENKELPROTHESE**
PROCEDURE FOR CONTROLLING A TRANSTIBIAL PROSTHESIS AND TRANSTIBIAL PROSTHESIS
PROCÉDÉ DE COMMANDE D'UNE PROTHÈSE DE JAMBE ET PROTHÈSE DE JAMBE

(30) Priorität: 10.10.2022 DE 102022126221
(43) Veröffentlichungstag der Anmeldung: 20.08.2025
(73) Patentinhaber: Otto Bock Healthcare Products GmbH, 1110 Wien (AT)
(72) Erfinder: BOHLAND, Andreas, 1110 Wien (AT)
(74) Vertreter: Gramm, Lins & Partner Patent- und Rechtsanwälte PartGmbB
(86) Internationale Anmeldenummer: PCT/EP2023/077905
(87) Internationale Veröffentlichungsnummer: WO 2024/079051

(56) Entgegenhaltungen:
- EP-B1- 2 124 842
- US-A1- 2012 232 673
- US-A1- 2019 142 611
- US-B1- 10 980 648

## Beschreibung

Die Erfindung betrifft ein Verfahren zum Steuern einer Unterschenkelprothese, die ein Fußelement, ein schwenkbar daran angeordnetes Unterschenkelelement und eine verstellbare Widerstandseinrichtung zum Aufbringen eines Widerstandes gegen eine Verschwenkung des Fußelementes relativ zu dem Unterschenkelelement aufweist, wobei bei dem Verfahren die Geschwindigkeit der Fortbewegung des Benutzers der Unterschenkelprothese ermittelt wird. Die Erfindung betrifft zudem eine Unterschenkelprothese zum Durchführen eines derartigen Verfahrens.

Unterschenkelprothesen, die mit einem hier beschriebenen Verfahren gesteuert werden, sind aus dem Stand der Technik, beispielsweise der US 10 980 648 B1, seit langem bekannt.

Weitere Knöchelprothesen mit variabler Widerstandseinrichtung und Verfahren zu deren Steuerung sind beispielsweise aus der EP 2 124 842 B1, der US 2019/0142611 A1 und der US 2012/0232673 A1 bekannt.

Während der Standphase eines Schrittzyklus, also der Zeit, in der das Fußelement Kontakt mit dem Boden hat, kommt es zum Überrollen des Unterschenkelelementes, also zu einer Verschwenkung des Unterschenkelelementes relativ zum Fußelement. Mit dem Fersenauftritt beginnt zunächst eine Plantarflexion, in der die Kontaktfläche, mit der das Fußelement Kontakt mit dem Boden hat, zunimmt, bis das Fußelement vollflächig, also mit der ganzen Sohle, Kontakt mit dem Boden hat. Der Winkel zwischen dem Unterschenkelelement und dem Fußelement wird dabei größer, sodass von einer Plantarflexion gesprochen wird.

An diese Phase schließt sich eine Dorsalflexion, also die Bewegung in die entgegengesetzte Richtung an. **In** dieser Phase bewegt sich der Schwerpunkt des Körpers des Trägers über das Fußelement hinweg und der Winkel zwischen dem Unterschenkelelement und dem Fußelement wird kleiner. Dieser Vorgang heißt Dorsalflexion und wird auch mit "überrollen" beschrieben. Ein gesunder menschlicher Fuß führt diese beiden Bewegungen während der Standphase eines Schrittzyklus ebenfalls aus. Der Schrittzyklus endet bei einem gesunden Fuß mit einer aktiven Plantarflexion, also einer aktiv hervorgerufenen Vergrößerung des Winkels zwischen dem Unterschenkel und dem Fuß. **In** dieser Bewegungsphase stößt der Fuß sich und damit den Menschen, um dessen Fuß es sich handelt, ab und unterstützt so die Vorwärtsbewegung.

Bei einer Unterschenkelprothese ist diese aktive Plantarflexion oftmals nicht möglich oder nicht ausreichend stark, sodass der Träger der Prothese die Vorwärtsbewegung auf andere Weise unterstützen muss, was oftmals zu einem unnatürlichen Gangbild führt. Das Fußelement verfügt daher in der Regel über wenigstens ein Federelement, beispielsweise eine Blattfeder aus einem faserverstärkten Kunststoff, beispielsweise einem Carbonfaser-verstärkten Kunststoff. Dieses Federelement soll während der letzten Phase des Schrittzyklus elastisch verformt werden und die dadurch in ihm gespeicherte Energie am Ende der Standphase abgeben und so den Fuß nach vorne beschleunigen. Damit dies geschieht, muss der Widerstand, den die Widerstandseinrichtung der Verschwenkung des Fußelementes relativ zum Unterschenkelelement entgegenstellt, erhöht werden. Dies kann kontinuierlich oder in Form eines plötzlichen Schaltens geschehen. Der Zeitpunkt, zu dem der Widerstand seinen Maximalwert erreicht, wird Maximalzeitpunkt genannt.

**In** vielen Ausgestaltungen ist der Widerstand, der zum Maximalzeitpunkt erreicht wird, so groß, dass eine weitere Verschwenkung des Unterschenkelelementes relativ zum Fußelement nicht mehr möglich ist. Dies ist zwar von Vorteil, jedoch nicht zwingend notwendig.

Das Ziel der Steuerung der Unterschenkelprothese ist es unter anderem, ein möglichst naturnahes Gangbild bei unterschiedlichen Geschwindigkeiten der Fortbewegung des Benutzers der Unterschenkelprothese zu erreichen. Dabei muss berücksichtigt werden, dass die in dem Federelement des Fußelementes gespeicherte Energie, die für ein natürliches Gangbild benötigt wird, von verschiedenen Parametern der Bewegung, beispielsweise der Geschwindigkeit der Fortbewegung des Benutzers abhängt. Aus dem Stand der Technik sind unterschiedliche Ansätze bekannt, dies umzusetzen. Es ist beispielsweise bekannt, den Widerstand, der durch die Widerstandseinrichtung dem Verschwenken entgegengesetzt wird in Abhängigkeit der detektiert Geschwindigkeit der Fortbewegung zu steuern, wobei insgesamt der Widerstand reduziert wird, je schneller sich der Träger der Unterschenkelprothese bewegt. Dahinter steckt die Idee, dass langsames Gehen dem Stehen ähnlich ist und daher eine Stabilisierung des Knöchelgelenkes notwendig und sinnvoll ist. Dies ist insbesondere bei Unterschenkelprothesen von Vorteil, deren Fußelement kein Federelement aufweist.

Aus der EP 3 427 701 B1 hingegen ist es bekannt, den Maximalzeitpunkt von der ermittelten Geschwindigkeit der Fortbewegung abhängig zu machen. Je größer die Geschwindigkeit ist, desto später in der Standphase wird der Maximalzeitpunkt erreicht. Dadurch soll ein größerer Bewegungsbereich zwischen dem Unterschenkelelement und dem Fußelement erreicht werden, sodass es dem Träger der Unterschenkelprothese leichter fällt, sich schnell zu bewegen. Außerdem wird die Bewegung zwischen dem Unterschenkelelement und dem Fußelement über einen größeren Zeitraum, nämlich bis zum Erreichen des Maximalzeitpunktes, vereinfacht und erleichtert.

Ein weiterer technischer Grund dafür, den Maximalzeitpunkt mit höherer Geschwindigkeit in der Standphase nach hinten zu verschieben besteht darin, dass oftmals ein hydraulisches System in der Unterschenkelprothese enthalten ist und eine Bewegung des Unterschenkelelementes relativ zum Fußelement zur Folge hat, dass ein Hydraulikfluid aus einer Kammer des Systems in eine andere Kammer bewegt wird. Der dadurch der Bewegung entgegengesetzte Widerstand ist abhängig von der Viskosität des Hydraulikfluids, die wiederum von der Geschwindigkeit abhängt. Dabei nimmt die Viskosität mit steigender Geschwindigkeit ab, sodass der Strömungswiderstand ansteigt. Um dies zu kompensieren, wird der durch die Widerstandseinrichtung aufgebrachte Widerstand reduziert.

Nachteilig ist jedoch, dass insbesondere bei großen Geschwindigkeiten, mit denen der Benutzer der Unterschenkelprothese sich fortbewegt, die im Fußelement gespeicherte oder speicherbare Energiemenge für eine natürliche Bewegung und zur Aufrechterhaltung der Geschwindigkeit oftmals nicht ausreicht. Der Erfindung liegt daher die Aufgabe zugrunde, diesen Nachteil abzumildern oder vollständig zu beheben.

Die Erfindung löst die gestellte Aufgabe durch ein Verfahren zum Steuern einer Unterschenkelprothese, die ein Fußelement, ein schwenkbar daran angeordnetes Unterschenkelelement und eine verstellbare Widerstandseinrichtung zum Aufbringen eines Widerstandes gegen eine Verschwenkung des Fußelementes relativ zu dem Unterschenkelelement aufweist, wobei bei dem Verfahren
- die Geschwindigkeit der Fortbewegung des Benutzers der Unterschenkelprothese ermittelt wird,
- aus der ermittelten Geschwindigkeit ein Maximalzeitpunkt innerhalb der Standphase des Schrittzyklus bestimmt wird,
- bei Erreichen des Maximalzeitpunkt der Widerstand der Widerstandseinrichtung auf einen maximal wird erhöht wird,
- wobei der Maximalzeitpunkt umso früher in der Standphase liegt, je höher die ermittelte Geschwindigkeit ist.

Der Maximalzeitpunkt ist folglich eine Funktion der ermittelten Geschwindigkeit, mit der sich der Benutzer der Unterschenkelprothese bewegt. Mit wachsender Geschwindigkeit verschiebt sich der Maximalzeitpunkt innerhalb der Standphase nach vorn, also zu früheren Zeiten. Dies bedeutet, dass der Wert des Maximalzeitpunktes innerhalb der Standphase eine mit wachsender Geschwindigkeit monoton sinkende Funktion ist. Zumindest abschnittsweise ist der Wert des Maximalzeitpunktes innerhalb der Standphase eine mit wachsender Geschwindigkeit streng monoton sinkende Funktion. Vorzugsweise ist er über den gesamten Geschwindigkeitsbereich eine streng monoton sinkende Funktion. Eine sinkende Funktion in diesem Zusammenhang bedeutet, dass mit steigender Geschwindigkeit der Maximalzeitpunkt zu früheren Zeitpunkten innerhalb der Standphase verschoben wird.

Dass der Widerstand beim Erreichen des Maximalzeitpunktes auf den Maximalwert erhöht wird, bedeutet im Sinne der vorliegenden Erfindung, dass zu diesem Zeitpunkt der Widerstand angehoben wird, sofern der Widerstand einen kleineren Wert annimmt. Sollte der Widerstand zu diesem Zeitpunkt bereits den Maximalwert annehmen, wird keine Veränderung des Widerstandes notwendig und auch nicht durchgeführt.

**In** der Regel muss der Widerstand der Widerstandseinrichtung jedoch angehoben werden, um den Maximalwert zu erreichen. Dafür muss in der Regel die verstellbare Widerstandseinrichtung verstellt werden, damit der Widerstand angepasst wird. Dafür vergeht eine gewisse Zeitspanne, sodass in diesem Fall zum Maximalzeitpunkt selbst der Widerstand noch nicht den Maximalwert annimmt.

Je höher folglich die Geschwindigkeit ist, mit der sich der Benutzer der Unterschenkelprothese fortbewegt, desto früher wird das Verschwenken des Unterschenkelelementes relativ zum Fußelement erschwert, indem der Widerstand der Widerstandseinrichtung auf den Maximalwert erhöht wird. Dadurch wird erreicht, dass mit höherer Geschwindigkeit mehr Energie in einem elastischen Element des Fußelementes, beispielsweise einer Carbon-Feder, gespeichert und zu einem späteren Zeitpunkt wieder abgegeben und in Bewegungsenergie umgewandelt und dem Fuß zur Verfügung gestellt wird. Überraschenderweise wird dadurch der Bewegungsablauf der natürlichen Bewegung besser angeglichen.

Die Unterschenkelprothese verfügt über eine elektrische Steuerung, die eingerichtet ist, die Verfahrensschritte durchzuführen.

Vorzugsweise entspricht der Maximalzeitpunkt einem Schaltwinkel zwischen dem Fußelement und dem Unterschenkelelement und/oder einem Absolutwinkel des Unterschenkelelementes in der Dorsalflexionsphase der Standphase des Schrittzyklus, wobei der Schaltwinkel umso größer ist, je größer die ermittelte Geschwindigkeit ist. **In** der Dorsalflexionsphase nimmt der Winkel zwischen dem Unterschenkelelement und dem Fußelement stetig ab. Je größer also der Schaltwinkel ist, desto früher in der Standphase erreicht der Widerstand den Maximalwert. Der "Zeitpunkt", zu dem der Maximalzeitpunkt erreicht ist, wird also in diesem Fall nicht in Sekunden oder einer anderen Zeiteinheit gemessen, sondern entspricht einem Winkel, der während der Standphase erreicht wird. Dieser Winkel ist beispielsweise ein Winkel zwischen dem Unterschenkelelement und dem Fußelement. Sobald also dieser Winkel, der auch Knöchelwinkel genannt wird, einen vorbestimmten Wert erreicht, gilt der Maximalzeitpunkt als erreicht und der Widerstand wird auf den Maximalwert erhöht. Wird der Knöchelwinkel als Kriterium für das Erreichen des Maximalzeitpunkt verwendet, ist es unerheblich, nach wie viel Sekunden dieser Knöchelwinkel erreicht wird. Ein größerer Knöchelwinkel gilt als früherer Maximalzeitpunkt.

Alternativ oder zusätzlich dazu wird ein Absolutwinkel des Unterschenkelelementes als Schaltwinkel verwendet. Dieser kann über Absolutwinkelsensoren ermittelt werden. Vorzugsweise weist ein solcher Sensor eine Inertialmesseinheit (IMU inertial measurement unit) auf. Alternativ oder zusätzlich dazu verfügt der Sensor über eine Kombination aus Accelerometern und/oder Gyroskopen, aus der die Orientierung des Unterschenkelelementes im Raum und daraus der Absolutwinkel bestimmt wird. Alternativ oder zusätzlich dazu verfügt die Unterschenkelprothese über wenigstens einen Sensor, der eingerichtet ist, den Absolutwinkel des Fußelementes und einen Relativwinkel zwischen Fußelement und Unterschenkelelement zu bestimmen. Aus der Kombination dieser beiden Winkel wird vorzugsweise der Absolutwinkel des Unterschenkelelementes bestimmt.

Der so ermittelte Wert wird mit einem für den Schaltwinkel festgelegten Wert verglichen, sodass innerhalb von der elektrischen Steuerung mittels eines Algorithmus entschieden werden kann, ob der Schaltwinkel erreicht wurde. Ein Absolutwinkel gibt Auskunft über die absolute Lage eines Gegenstandes, vorliegend also des Unterschenkelelementes, im Raum. Er wird gegen eine festgelegte Basis-Richtung gemessen. Ist ein Gegenstand beispielsweise vertikal ausgerichtet, folgt seine Längsrichtung also der durch die Schwerkraft vorgegebenen Richtung, hat er einen Absolutwinkel von 0°, wenn die von der Schwerkraft vorgegebenen Richtung, also die Vertikale, die Basis-Richtung ist. Ist hingegen die Basis-Richtung die Horizontale, die senkrecht auf der Vertikalen steht, ist bei einer vertikalen Ausrichtung des Gegenstandes auch der Absolutwinkel 90°.

**In** einer bevorzugten Ausgestaltung wird die Geschwindigkeit der Fortbewegung ermittelt, indem eine Schrittlänge und oder eine Schrittdauer ermittelt wird, wobei die ermittelte Schrittlänge und/oder die ermittelte Schrittdauer einer Geschwindigkeit zugeordnet wird. Dabei ist die zugeordnete Geschwindigkeit umso größer, je größer die ermittelte Schrittlänge und/oder je kleiner die Schrittdauer ist. Anstelle der Schrittdauer wird oft die sogenannte Kadenz, also der Kehrwert der Schrittdauer, der in Hertz angegeben wird, verwendet. Auch dies gilt im Rahmen der vorliegenden Erfindung als Verwendung der Schrittdauer.

**In** einer bevorzugten Ausgestaltung wird zur Bestimmung der Schrittlänge der Absolutwinkel des Unterschenkelelementes zum Zeitpunkt des Fersenauftrittes gemessen, wobei dem gemessenen Absolutwinkel eine Schrittlänge und damit eine Geschwindigkeit zugeordnet wird.

Vorzugsweise wird ein Winkelbereich ermittelt, den der Absolutwinkel des Unterschenkelelementes während einer Schwungphase eines Schrittzyklus überstreicht, wobei aus diesem ermittelten Winkelbereich die Schrittlänge bestimmt wird. Der Winkelbereich kann beispielsweise ermittelt werden, indem der Absolutwinkel des Unterschenkelelementes zum Zeitpunkt des Fersenauftrittes und zum Zeitpunkt des sogenannten "toe-off", also dem Zeitpunkt, an dem das Fußelement den Kontakt zum Boden verliert, bestimmt wird. Aus diesen beiden Messungen kann die Differenz gebildet und so der Winkelbereich ermittelt werden.

Vorzugsweise wird eine Winkelgeschwindigkeit des Absolutwinkels des Unterschenkelelementes in der Schwungphase des Schrittzyklus gemessen. Dabei wird aus dieser Winkelgeschwindigkeit die Geschwindigkeit der Fortbewegung bestimmt oder der Winkelgeschwindigkeit eine Geschwindigkeit zugeordnet. Bei der Winkelgeschwindigkeit handelt es sich um die erste Ableitung des Absolutwinkels des Unterschenkelelementes nach der Zeit. Es geht also um die Änderung des Absolutwinkels in einer vorgegebenen Zeiteinheit. Je größer diese Ableitung ist, desto größer ist auch die Geschwindigkeit der Fortbewegung, die aus dieser Änderung bestimmt oder ihr zugeordnet wird. Alternativ oder zusätzlich dazu wird die Geschwindigkeit der Fortbewegung mittels wenigstens einem Gyroskop, vorzugsweise mehreren Gyroskopen gemessen.

Vorzugsweise wird der Widerstand der Widerstandseinrichtung erst in der Standphase des folgenden Schrittzyklus wieder abgesenkt. Besonders bevorzugt erfolgt dieser Absenkung mit dem Fersenauftritt im folgenden Schrittzyklus. Dies hat zur Folge, dass auch in der zwischen den beiden aufeinanderfolgenden Standphasen liegenden Schwungphase der Widerstand der Widerstandseinrichtung auf dem Maximalwert bleibt und so eine Verschwenkung des Unterschenkelelementes relativ zum Fußelement erschwert oder vollständig unmöglich ist. Die Unterschenkelprothese verbleibt folglich in der Schwungphase in der zum Maximalzeitpunkt eingenommenen Position. Dies bedeutet, Unterschenkelelement und Fußelement bleiben nahezu oder vollständig unverändert zueinander orientiert. Dies ist insbesondere dann von Vorteil, wenn der Maximalzeitpunkt erreicht ist, wenn der Winkel zwischen dem Unterschenkelelement und dem Fußelement kleiner als 90° ist. Die Richtung des Unterschenkelelementes entspricht dabei der Längsrichtung des als Unterschenkelelement verwendeten Prothesenbauteils. Die Richtung des Fußelementes entspricht der Richtung der Trittfläche des Fußelementes, also der Fläche, die mit dem Boden in Kontakt gebracht werden kann. Je weiter dorsal reflektiert der Knöchelwinkel ist, also je weiter der Vorfuß angehoben ist, desto stärker wird durch die Aufrechterhaltung des Maximalwertes des Widerstandes ein Stolpern während der Schwungphase verhindert.

**In** einer bevorzugten Ausgestaltung wird der Widerstand nach dem Erreichen des Maximalzeitpunkt auf einem Wert gehalten, der so hoch ist, dass eine weitere Verschwenkung des Fußelementes relativ zu dem Unterschenkelelement nicht mehr möglich ist, sofern die ermittelte Geschwindigkeit einen vorbestimmten Grenzwert überschreitet. Besonders bevorzugt wird der Widerstand erst dann wieder abgesenkt, wenn die ermittelte Geschwindigkeit den vorbestimmten Grenzwert wieder unterschreitet. Dies hat zur Folge, dass beispielsweise beim schnellen Laufen des Trägers der Unterschenkelprothese der Widerstand dauerhaft so hoch ist, dass das Fußelement nicht relativ zum Unterschenkelelement bewegt werden kann. Dies hat zur Folge, dass eine Standphase nicht mit einem Fersenauftritt beginnt, sondern bereits zu diesem frühen Zeitpunkt der Standphase ein Kontakt zwischen dem Vorfuß des Fußelementes und dem Boden zustande kommt. Dies entspricht dem natürlichen Bewegungsablauf eines gesunden Menschen, der beim schnellen Laufen nur mit dem Vorfußbereich, im Wesentlichen also den Zehen, den Boden berührt.

Die Erfindung löst die gestellte Aufgabe zudem durch eine Unterschenkelprothese mit einem Fußelement, einem schwenkbar daran angeordneten Unterschenkelelement und einer verstellbaren Widerstandseinrichtung zum Aufbringen eines Widerstandes gegen eine Verschwenkung des Fußelementes relativ zu dem Unterschenkelelement, wobei die Unterschenkelprothese wenigstens einen Sensor zum Bestimmen von Messdaten und eine elektrische Steuerung aufweist, die eingerichtet ist, aus den Messdaten die Geschwindigkeit der Fortbewegung zu ermitteln und ein Verfahren nach einem der vorstehenden Ansprüche durchzuführen. Die Unterschenkelprothese weist eine elektrische Steuerung, vorzugsweise in Form einer elektronischen Datenverarbeitungseinrichtung auf, die eingerichtet ist, eines der oben genannten Verfahren durchzuführen.

Vorzugsweise weist die Widerstandseinrichtung ein Hydrauliksystem auf. Dieses beinhaltet ein Hydraulikfluid, das bei der Bewegung des Unterschenkelelementes relativ zu dem Fußelement innerhalb des Hydrauliksystems bewegt wird. Dabei setzt das Hydrauliksystem der Bewegung des Fluids einen Strömungswiderstand entgegen. Dieser Strömungswiderstand ist über ein Stellglied einstellbar. Ein solches Stellglied ist beispielsweise als Drosselventil ausgebildet, dessen Strömungsquerschnitt einstellbar ist.

Alternativ oder zusätzlich dazu weist die Widerstandseinrichtung eine magnetorheologische Einrichtung und/oder einen Elektromotor auf.

Mit Hilfe der beigefügten Darstellungen wird eine Ausführungsform der vorliegenden Erfindung näher erläutert. Es zeigen:
Figuren 1 bis 3 - verschiedene Phasen in einem Schrittzyklus bei unterschiedlichen Geschwindigkeiten der Fortbewegung.

Die Figuren 1 bis 3 zeigen jeweils fünf Darstellungen unterschiedlicher Phasen in einem Schrittzyklus. Von links nach rechts wird jeweils das Ende der Schwungphase ("Terminal Swing"), die Belastungsreaktion ("Loading Response"), die mittlere Standphase ("MidStance"), das Ende der Standphase ("Terminal Stance") und der Beginn der Schwungphase ("Initial Swing") dargestellt. Figur 1 zeigt den Ablauf beim langsamen Gehen, Figur 2 beim schnellen Gehen und Figur 3 beim Laufen oder Rennen.

**In** den Figuren ist jeweils eine Unterschenkelprothese mit einem Unterschenkelelement 2 und einem Fußelement 4 gezeigt, die um eine Schwenkachse schwenkbar aneinander angeordnet sind. Die Unterschenkelprothese verfügt über eine nicht dargestellte Widerstandseinrichtung, durch die ein Widerstand dem Verschwenken der beiden Elemente 2,4 relativ zueinander entgegengesetzt werden kann. Dieser Widerstand ist durch die Widerstandseinrichtung einstellbar.

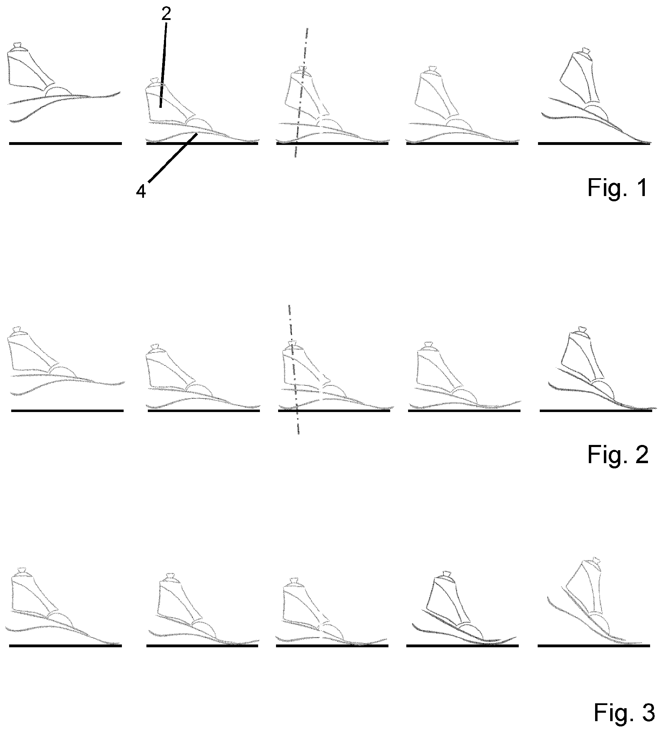

**In** Figur 1 ist der Ablauf beim langsamen Gehen dargestellt. Am Ende der Schwungphase (Darstellung ganz links) ist die Widerstandseinrichtung so eingestellt, dass ein Widerstand gegen das Verschwenken des Fußelementes 4 relativ zum Unterschenkelelement 2 gering ist. **In** einer bevorzugten Ausführungsform wird in dieser Situation nur ein geringer Widerstand durch die Widerstandseinrichtung aufgebracht. Bei der Belastungsreaktion (zweite Darstellung von links) hat der Knöchel einen hohen Bewegungsumfang. Das Unterschenkelelement 2 ist in der gezeigten Darstellung weit plantarflektiert, in der gezeigten Darstellung also gegen den Uhrzeigersinn relativ zum Fußelement 4 verschwenkt. Man erkennt, dass das Unterschenkelelement 2 sehr nah an einer oberen Feder des Fußelementes 4 dargestellt ist. Eine ebenfalls auftretende Verformung der Federelemente auf der Ferse des Fußelementes 4 ist in keiner der Darstellungen gezeigt. Im weiteren Verlauf der Standphase (mittlere Darstellung in Figur 1) wird das Unterschenkelelement 2 relativ zum Fußelement 4 dorsalflektiert. Erst zu einem relativ späten Zeitpunkt in dieser Bewegung wird der Maximalzeitpunkt erreicht, ab dem der Widerstand, der durch die Widerstandseinrichtung hervorgerufen wird, soweit erhöht wird, dass im gezeigten Ausführungsbeispiel eine weitere Verschwenkung des Unterschenkelelementes 2 relativ zum Fußelement 4 nicht mehr möglich ist. Im langsamen Gehen (Figur 1) geschieht dies erst gegen Ende der Standphase (zweite Darstellung von rechts). Man erkennt, dass bis zu diesem Zeitpunkt ein Winkel zwischen dem Unterschenkelelement 2 und dem Fußelement 4 immer weiter abnimmt, was gleichbedeutend ist mit einem zunehmendem Abstand zwischen dem Unterschenkelelement 2 und der oberen Feder des Fußelementes 4 im Bereich der Ferse. Da der Maximalzeitpunkt sehr spät erreicht wird oder in einer bevorzugten Ausgestaltung das Unterschenkelelement 2 bis zum mechanischen Anschlag relativ zum Fußelement 4 verschwenkt werden kann, ist die Bewegung nach diesem Zeitpunkt nur noch gering, sodass auch die Federn des Fußelementes 4 nur wenig gespannt werden können. Daher kann in der beginnenden Schwungphase (Darstellung ganz rechts) nur wenig Energie abgegeben werden. Während der Schwungphase wird keine Energie mehr abgegeben und eine Bewegung des Unterschenkelelementes 2 relativ zum Fußelement 4 findet nicht oder nicht in nennenswertem Umfang statt. Die Position des Unterschenkelelementes 2 relativ zum Fußelement 4 in dieser Phase entspricht daher der Position, mit der der Beginn der nächsten Standphase (Darstellung ganz links) eingeleitet wird.

Figur 2 zeigt die gleichen Darstellungen für den Fall, dass der Träger der Prothese schnell geht. Man erkennt, dass der Beginn der Standphase (Darstellung ganz links) mit einem deutlich flacheren Fuß auftritt beginnt. Der Fuß ist stärker plantarflektiert als dies in Figur 1 beim langsamen Gehen der Fall ist. Diese Situation ergibt sich dann, wenn auch der vorhergehende Schritt bereits mit der höheren Geschwindigkeit durchgeführt wurde. Auch beim schnellen Gehen ist zunächst eine Bewegung des Unterschenkelelementes 2 relativ zum Fußelement 4 möglich. In der Belastungsreaktion (zweite Darstellung von links) unterscheidet sich - vereinfacht ausgedrückt - die Position des Unterschenkelelementes 2 relativ zum Fußelement 4 nicht von der beim langsamen Gehen. Beim Überrollen in der Mitte der Standphase (mittlere Darstellung) wird jedoch der Maximalzeitpunkt deutlich früher erreicht, als dies beim langsamen Gehen der Fall war. Daher blockiert die Widerstandseinrichtung ab diesem Zeitpunkt eine weitere Verschwenkung des Unterschenkelelementes 2 relativ zum Fußelement 4 durch den von ihr aufgebrachten Widerstand. Zumindest aber erschwert der aufgebracht Widerstand diese weitere Verschwenkung. Man erkennt daher, dass der Abstand zwischen dem Unterschenkelelement 2 und dem Fußelement 4 in der Mitte der Standphase beim schnellen Gehen deutlich kleiner ist als beim langsamen Gehen. Dies bedeutet, dass beim weiteren Überrollen die Federn des Fußelementes 4 verformt werden. Dies ist am Ende der Standphase (zweite Darstellung von rechts) deutlich zu erkennen. In den Federn wird folglich mehr potentielle Energie gespeichert, die zum Beginn der Schwungphase (Darstellung ganz rechts) abgegeben wird. Dies führt dazu, dass das Fußelement 4 relativ zum Unterschenkelelement 2 in einer deutlich stärker plantarflektierten Stellung gehalten wird als beim langsamen Gehen. Auch beim schnellen Gehen findet keine weitere Bewegung des Fußelementes 2 relativ zum Unterschenkelelement 4 während der Schwungphase statt, sodass auch hier die Position zu Beginn der Schwungphase (Darstellung ganz rechts) der Position am Ende der Schwungphase (Darstellung ganz links) entspricht.

Figur 3 zeigt die Situation beim Rennen. In der gezeigten Ausführungsform ist die Geschwindigkeit dabei so groß, dass der Maximalzeitpunkt sehr früh erreicht wurde. Wird über mehrere Schritte hinweg der frühestmögliche Maximalzeitpunkt erreicht, wird die Widerstandseinrichtung so gesteuert, dass der von ihr erzeugte Widerstand konstant hoch bleibt. Im gezeigten Ausführungsbeispiel hat dies zur Folge, dass eine Verschwenkung des Unterschenkelelementes 2 relativ zum Fußelement 4 nicht mehr möglich ist und sich daher die Orientierung und Position der beiden Elemente relativ zueinander nicht verändert. Sämtliche Abrollbewegungen werden daher durch die Verformung der Federn ermöglicht. Dies hat beispielsweise zur Folge, dass der Beginn der Standphase, also das Ende der Schwungphase (Darstellung ganz links) nicht mit einem Fersenauftritt, sondern mit einem Zehenauftritt beginnt. Über die gesamte Standphase hinweg (zweite Darstellung von links bis zweite Darstellung von rechts) kommt es nicht zu einem Kontakt zwischen der Ferse und dem Boden. Die gesamte Energie aus der Vorfußbelastung ist am Ende der Standphase (zweite Darstellung von rechts) in den verformten Federn gespeichert und wird zu Beginn der Schwungphase (Darstellung ganz rechts) abgegeben.

### Bezugszeichenliste:

Unterschenkelelement 2
Fußelement 4

## Patentansprüche

1. Verfahren zum Steuern einer Unterschenkelprothese, die ein Fußelement (4), ein schwenkbar daran angeordnetes Unterschenkelelement (2) und eine verstellbare Widerstandseinrichtung zum Aufbringen eines Widerstandes gegen eine Verschwenkung des Fußelementes (4) relativ zu dem Unterschenkelelement (2) aufweist, wobei bei dem Verfahren
- die Geschwindigkeit der Fortbewegung des Benutzers der Unterschenkelprothese ermittelt wird,
**dadurch gekennzeichnet, dass bei dem Verfahren**
- aus der ermittelten Geschwindigkeit ein Maximalzeitpunkt innerhalb der Standphase des Schrittzyklus bestimmt wird,
- bei Erreichen des Maximalzeitpunktes der Widerstand der Widerstandseinrichtung auf einen Maximalwert erhöht wird,
- wobei der Maximalzeitpunkt umso früher in der Standphase liegt, je höher die ermittelte Geschwindigkeit ist.

2. Verfahren nach Anspruch 1, **dadurch gekennzeichnet, dass** der Maximalzeitpunkt einem Schaltwinkel zwischen dem Fußelement (4) und dem Unterschenkelelement (2) und/oder einem Absolutwinkel des Unterschenkelelementes (2) in der Dorsalflexionsphase der Standphase des Schrittzyklus entspricht und der Schaltwinkel umso größer ist, je größer die ermittelte Geschwindigkeit ist.

3. Verfahren nach Anspruch 1 oder 2, **dadurch gekennzeichnet, dass** die Geschwindigkeit der Fortbewegung ermittelt wird, indem eine Schrittlänge und/oder die Schrittdauer ermittelt wird, und die ermittelte Schrittlänge und/oder Schrittdauer einer Geschwindigkeit zugeordnet wird, wobei die zugeordnete Geschwindigkeit umso größer ist, je größer die ermittelte Schrittlänge und/oder je kleiner die Schrittdauer ist.

4. Verfahren nach Anspruch 3, **dadurch gekennzeichnet, dass** ein Absolutwinkel des Unterschenkelelementes (2) zum Zeitpunkt des Fersenauftrittes gemessen und aus diesem Absolutwinkel die Schrittlänge bestimmt wird.

5. Verfahren nach Anspruch 3 oder 4, **dadurch gekennzeichnet, dass** ein Winkelbereich ermittelt wird, den der Absolutwinkel des Unterschenkelelementes (2) während einer Schwungphase eines Schrittzyklus überstreicht, und dass aus diesem Winkelbereich die Schrittlänge bestimmt wird.

6. Verfahren nach einem der vorstehenden Ansprüche, **dadurch gekennzeichnet, dass** eine Winkelgeschwindigkeit des Absolutwinkels des Unterschenkelelementes (2) in einer Schwungphase eines Schrittzyklus gemessen und daraus die Geschwindigkeit der Fortbewegung bestimmt wird.

7. Verfahren nach einem der vorstehenden Ansprüche, **dadurch gekennzeichnet, dass** der Widerstand der Widerstandseinrichtung erst in der Standphase des folgenden Schrittzyklus wieder abgesenkt wird.

8. Verfahren nach einem der vorstehenden Ansprüche, **dadurch gekennzeichnet, dass** der Widerstand nach dem Erreichen des Maximalzeitpunktes so hoch gehalten bleibt, dass eine weitere Verschwenkung des Fußelementes (4) relativ zu dem Unterschenkelelement (2) nicht mehr möglich ist, wenn die ermittelte Geschwindigkeit einen vorbestimmten Grenzwert überschreitet.

9. Verfahren nach Anspruch 8, **dadurch gekennzeichnet, dass** der Widerstand erst dann wieder abgesenkt wird, wenn die ermittelte Geschwindigkeit den vorbestimmten Grenzwert wieder unterschreitet.

10. Unterschenkelprothese mit einem Fußelement (4), einem schwenkbar daran angeordneten Unterschenkelelement (2)und einer verstellbaren Widerstandseinrichtung zum Aufbringen eines Widerstandes gegen eine Verschwenkung des Fußelementes (4) relativ zu dem Unterschenkelelement (2), wobei die Unterschenkelprothese wenigstens einen Sensor zum Bestimmen von Messdaten und eine elektrische Steuerung aufweist, die eingerichtet ist, aus den Messdaten die Geschwindigkeit der Fortbewegung zu ermitteln, wobei die elektrische Steuerung eingerichtet ist, ein Verfahren nach einem der vorstehenden Ansprüche durchzuführen.

11. Unterschenkelprothese nach Anspruch 10, **dadurch gekennzeichnet, dass** die Widerstandseinrichtung ein Hydrauliksystem aufweist.

12. Unterschenkelprothese nach Anspruch 10 oder 11, **dadurch gekennzeichnet, dass** die Widerstandseinrichtung eine magnetorheologische Einrichtung aufweist.

13. Unterschenkelprothese nach einem der Ansprüche 10 bis 12, **dadurch gekennzeichnet, dass** die Widerstandseinrichtung einen Elektromotor aufweist.

## Claims

1. A method for controlling a lower leg prosthesis comprising a foot element (4), a lower leg element (2) pivotably arranged thereon and an adjustable resistance device for applying a resistance against a swivelling of the foot element (4) relative to the lower leg element (2), the method comprising the following step:
- identifying the speed of locomotion of the user of the lower leg prosthesis,
**characterized by** the following steps:
- determining a maximum point within the stance phase of the step cycle from the identified speed,
- increasing the resistance of the resistance device to a maximum when the maximum point is reached,
- wherein the higher the identified speed, the earlier the maximum point is in the stance phase.

2. The method according to claim 1, **characterized in that** the maximum point corresponds to a switching angle between the foot element (4) and the lower leg element (2) and/or an absolute angle of the lower leg element (2) in the dorsal flexion phase of the stance phase of the step cycle, wherein the switching angle is greater the greater the identified speed.

3. The method according to claim 1 or 2, **characterized in that** the speed of locomotion is determined by identifying a step length and/or step duration, and the identified step length and/or identified step duration is allocated to a speed, wherein the greater the identified step length and/or the shorter the step duration, the greater the speed allocated.

4. The method according to claim 3, **characterized in that** an absolute angle of the lower leg element (2) is measured at the point of heel strike and the step length is determined from said absolute angle.

5. The method according to claim 3 or 4, **characterized in that** an angular range is determined which the absolute angle of the lower leg element (2) covers during a swing phase of a step cycle, and that the step length is determined from this angular range.

6. The method according to one of the preceding claims, **characterized in that** an angular speed of the absolute angle of the lower leg element (2) is measured in a swing phase of a step cycle and the speed of locomotion is determined from said angular speed.

7. The method according to one of the preceding claims, **characterized in that** the resistance of the resistance device is only decreased again in the stance phase of the following step cycle.

8. The method according to one of the preceding claims, **characterized in that**, upon reaching the maximum point, the resistance is kept so high that a further swivelling of the foot element (4) relative to the lower leg element (2) is no longer possible if the speed determined exceeds a predetermined limit value.

9. The method according to claim 8, **characterized in that** the resistance does not decrease again until the speed determined falls back below the predetermined limit value.

10. A lower leg prosthesis with a foot element (4), a lower leg element (2) pivotably arranged thereon and an adjustable resistance device for applying a resistance against a swivelling of the foot element (4) relative to the lower leg element (2), wherein the lower leg prosthesis comprises at least one sensor for determining measurement data and an electrical control unit that is configured to determine the speed of locomotion from the measurement data wherein the electrical control is configured to conduct a method according to one of the preceding claims.

11. The lower leg prosthesis according to claim 10, **characterized in that** the resistance device has a hydraulic system.

12. The lower leg prosthesis according to claim 10 or 11, **characterized in that** the resistance device has a magnetorheological device.

13. The lower leg prosthesis according to one of the claims 10 to 12, **characterized in that** the resistance device has an electric motor.

## Revendications

1. Procédé de commande d'une prothèse de jambe comprenant un élément de pied (4), un élément de jambe (2) monté de manière pivotante sur celui-ci et un dispositif de résistance réglable destiné à exercer une résistance au pivotement de l'élément de pied (4) par rapport à l'élément de jambe (2), procédé dans lequel
- la vitesse de locomotion de l'utilisateur de la prothèse de jambe est déterminée,
**caractérisé en ce que**, dans le procédé
- un instant maximal pendant la phase d'appui du cycle de marche est défini à partir de la vitesse déterminée,
- lorsque l'instant maximal est atteint, la résistance du dispositif de résistance est augmentée à une valeur maximale,
- l'instant maximal se situant d'autant plus tôt dans la phase d'appui que la vitesse déterminée est élevée.

2. Procédé selon la revendication 1,
**caractérisé en ce que** l'instant maximal correspond à un angle de commutation entre l'élément de pied (4) et l'élément de jambe (2) et/ou à un angle absolu de l'élément de jambe (2) dans la phase de flexion dorsale de la phase d'appui du cycle de marche, et l'angle de commutation est d'autant plus grand que la vitesse déterminée est élevée.

3. Procédé selon la revendication 1 ou 2,
**caractérisé en ce que** la vitesse de locomotion est déterminée en déterminant une longueur de pas et/ou la durée du pas, et la longueur de pas et/ou la durée du pas déterminée est associée à une vitesse, la vitesse associée étant d'autant plus élevée que la longueur de pas déterminée est grande et/ou que la durée du pas est petite.

4. Procédé selon la revendication 3,
**caractérisé en ce qu'**un angle absolu de l'élément de jambe (2) est mesuré à l'instant où le talon touche le sol, et la longueur de pas est déterminée à partir de cet angle absolu.

5. Procédé selon la revendication 3 ou 4,
**caractérisé en ce qu'**une plage angulaire est déterminée, laquelle est balayée par l'angle absolu de l'élément de jambe (2) pendant une phase d'oscillation d'un cycle de marche, et **en ce que** la longueur de pas est définie à partir de cette plage angulaire.

6. Procédé selon l'une des revendications précédentes,
**caractérisé en ce qu'**une vitesse angulaire de l'angle absolu de l'élément de jambe (2) pendant une phase d'oscillation d'un cycle de marche est mesurée, et la vitesse de locomotion est définie à partir de celle-ci.

7. Procédé selon l'une des revendications précédentes,
**caractérisé en ce que** la résistance du dispositif de résistance n'est réduite que dans la phase d'appui du cycle de marche suivant.

8. Procédé selon l'une des revendications précédentes,
**caractérisé en ce que** la résistance, après avoir atteint l'instant maximal, reste suffisamment élevée pour qu'un pivotement supplémentaire de l'élément de pied (4) par rapport à l'élément de jambe (2) ne soit plus possible si la vitesse déterminée est supérieure à une valeur limite prédéfinie.

9. Procédé selon la revendication 8,
**caractérisé en ce que** la résistance n'est réduite que si la vitesse déterminée est à nouveau inférieure à la valeur limite prédéfinie.

10. Prothèse de jambe comprenant un élément de pied (4), un élément de jambe (2) monté de manière pivotante sur celui-ci et un dispositif de résistance réglable destiné à exercer une résistance au pivotement de l'élément de pied (4) par rapport à l'élément de jambe (2), la prothèse de jambe comportant au moins un capteur pour déterminer des données de mesure et une commande électrique qui est conçue pour déterminer la vitesse de locomotion à partir des données de mesure,
la commande électrique étant conçue pour mettre en œuvre un procédé selon l'une des revendications précédentes.

11. Prothèse de jambe selon la revendication 10,
**caractérisée en ce que** le dispositif de résistance comprend un système hydraulique.

12. Prothèse de jambe selon la revendication 10 ou 11,
**caractérisée en ce que** le dispositif de résistance comprend un dispositif magnétorhéologique.

13. Prothèse de jambe selon l'une des revendications 10 à 12,
**caractérisée en ce que** le dispositif de résistance comprend un moteur électrique.
